# EUROPEAN PATENT SPECIFICATION

(11) **EP 1 065 276 B1**
(45) Date of publication and mention of the grant of the patent: **29.09.2004**
(21) Application number: 00112724.0
(22) Date of filing: 15.06.2000
(51) Int. Cl.: C12N 15/53, C12P 7/18, C12P 19/02, C12N 1/16, C12N 1/21, C12N 9/04

(54) **Methods for producing D-arabitol, D-xylulose and xylitol using the yeast Metschnikowia**
Verfahren zur Herstellung von D-arabitol, D-xylulose und Xylitol durch Metschnikowia Hefe
Procédés pour la production de D-arabitol, D-xylulose et xylitol utilisant la levure Metschnikowia

(30) Priority: 24.06.1999 JP 17851499
(43) Date of publication of application: 03.01.2001
(73) Proprietor: Ajinomoto Co., Inc., Tokyo 104 (JP)
(72) Inventor: Nozaki, Hiroyuki, c/o Ajinomoto Co., Inc., Kawasaki-shi, Kanagawa (JP); Suzuki, Shunichi, c/o Ajinomoto Co., Inc., Kawasaki-shi, Kanagawa (JP); Takeuchi, Sonoko, c/o Ajinomoto Co., Inc., Kawasaki-shi, Kanagawa (JP); Tsuyoshi, Naoko, c/o Ajinomoto Co., Inc., Kawasaki-ku Kawasaki-shi, Kanagawa (JP); Yokozeki, Kenzo, c/o Ajinomoto Co., Inc., Kawasaki-shi, Kanagawa (JP)
(74) Representative: Strehl Schübel-Hopf & Partner

(56) References cited:
- EP-A- 0 403 392
- WO-A-99/20782
- SPONHOLZ W R ET AL: "THE FORMATION OF ALDITOLS BY THE YEAST OF WINE" CHEMIE MIKROBIOLOGIE TECHNOLOGIE DER LEBENSMITTEL, vol. 10, no. 1-2, 1986, pages 19-24, XP000946973 ISSN: 0366-7154
- PATENT ABSTRACTS OF JAPAN vol. 1999, no. 14, 22 December 1999 (1999-12-22) & JP 11 239496 A (AJINOMOTO CO INC), 7 September 1999 (1999-09-07)

### SEQUENCE LISTING

<110> Ajinomoto Co., Inc.
<120> Methods for producing D-arabitol, D-xylulose and'xylitol
<130> EPA-53408
<141> 2000-06-15
<150> JP 11-178514
   <151> 1999-06-24
<160> 2
<170> PatentIn Ver. 2.0
<210> 1
   <211> 26
   <212> DNA
   <213> Artificial Sequence
<220>
   <223> Description of Artificial Sequence: primer
<400> 1
   cgggaattcg atatcatttt aatgaa 26
<210> 2
   <211> 29
   <212> DNA
   <213> Artificial Sequence
<220>
   <223> Description of Artificial Sequence: primer
<400> 2
   ggcggatccg cagtcaatac cggcataga 29

## Description

### Field of the Invention

The present invention relates to methods for producing D-arabitol, D-xylulose and xylitol. D-arabitol and D-xylulose are useful as raw materials for the production of xylitol, and xylitol is useful in the fields of food industry, pharmaceutical industry and so forth.

### Related Art

Demand of xylitol, which is a naturally occurring saccharide alcohol, is expected to increase in future. Xylitol is a promising low-calorie sweetener because it has lower calories compared with sucrose but exhibits sweetness comparable to that of sucrose. In addition, because of its anti-dental caries property, it is used as a dental caries preventive sweetener. Furthermore, because xylitol does not elevate glucose level, it is used for fluid therapy in the treatment of diabetes mellitus.

The current industrial production of xylitol mainly relies on hydrogenation of D-xylose as disclosed in U.S. Patent No. 4,008,285. D-Xylose used as a raw material is obtained by hydrolysis of plant materials as starting materials such as trees, straws, corn cobs, oat hulls and other xylan-rich materials.

However, such D-xylose produced from hydrolysis of plant materials suffers from a drawback that it is rather expensive, and it is arisen from high production cost. For example, the low yield of the hydrolysis treatment of plant materials leads to low purity of the produced xylitol. Therefore, the acid used for the hydrolysis and the dyes must be removed by ion exchange treatment after the hydrolysis treatment, and the resulting D-xylose must be further crystallized to remove other hemicellulosic saccharides. In order to obtain D-xylose suitable for foodstuffs, further purification would be required. Such ion exchange treatment and crystallization treatment invite the increase of production cost.

In order to solve this problem, it has been desired to develop a method for producing xylitol that uses a readily available raw material and produce little waste. Therefore, there have been developed methods for producing xylitol utilizing other pentitols as a starting material. One of such readily available pentitols is D-arabitol.

Several methods for producing xylitol that utilize D-arabitol as a raw material have been developed. One method has been reported in *Applied Microbiology*, *18* (1969) 1031-1035, wherein D-arabitol is produced from glucose by fermentation using *Debaryomyces hansenii* ATCC 20121, and then converted into D-xylulose using *Acetobacter suboxydan*s, and the D-xylulose is converted into xylitol by the action of *Candida guilliermondii* var. soya.

Further, EP 403 392A (Applicant: Roquette Freres) and EP421 882A (Applicant: Roquette Freres) disclose methods which comprise producing D-arabitol by fermentation by using osmosis-resistant yeast, then converting the D-arabitol into D-xylulose by using a bacterium belonging to the genus *Acetobacter*, *Gluconobacter* or *Klebsiella*, forming a mixture of xylose and xylulose from the D-xylulose by the action of glucose (xylose) isomerase, and converting the produced mixture of xylose and xylulose into xylitol by hydrogenation. There is also disclosed the production of xylitol comprising preliminarily concentrating xylose in the mixture of xylose and xylulose and converting the concentrated xylose into xylitol by hydrogenation.

As for D-arabitol, on the other hand, it has been known that yeast strains belonging to the genera *Pichia*, *Hansenula*, *Debaryomyces, Zygosaccaromyces, Saccharomyces, Candida*, *Torulopsis* and so forth have the ability to produce D-arabitol. For example, Japanese Patent Publication (Kokoku) No. 47-20394/1972 discloses a method for converting a saccharide such as glycerol into D-arabitol by using a microorganism belonging to the genus *Candida*, *Saccharomyces* or *Torulopsis.* Further, Can. J. Microbiol. 31 (1985) 467-471 describes that cells of *Debaryomyces hansenii* accumulate arabitol therein in their stationary phase in a medium containing glucose, L-arabinose or the like, and J. Gen. Microbiol., 139 (1993) 1047-1054 describes that many yeast strains become to accumulate glycerol and D-arabitol, when they are applied with osmotic pressure by glucose.

By the way, in researches about production of alditols by wine yeast, it has been reported that several yeast strains produce D-arabitol when grape juice is used as a fermentation substrate (*Chem*. *Mikrobiol*. *Technol*. *Lebensm*., *10*, 19-24 (1986)). For example, it was confirmed that 5 g/L of D-arabitol was produced from 183 g/L of substrate glucose by *Metschnikowia pulcherrima*. The amount of the residual glucose in this case was 73 g/L, and therefore weight yield with respect to the consumed glucose was extremely low, i.e., about 2%. These researches did not aim at the production of D-arabitol first of all, and the detection was just qualitative for examining origin of alditols such as D-arabitol present in wine. Therefore, they did not disclose the concept of efficient production method of D-arabitol at all. This document discloses that Metschnikowia forms concentrations of arabitol of 1600 mg/l. Moreover, the fermentation of grape juice in the wine brewing is performed under a relatively anaerobic condition, which does not utilize aeration or stirring, and thus it has not been known that D-arabitol can efficiently be produced by culturing a yeast strain belonging to the genus *Metschnikowia* under an aerobic condition

### Summary of the Invention

An object of the present invention is to provide a method for efficiently producing D-arabitol, and methods for producing D-xylulose and xylitol by using the method

In order to achieve the aforementioned object, the inventors of the present invention tested various microorganisms for D-arabitol producing ability and conducted experiments about production conditions. As a result, they found that, when a microorganism belonging to the genus *Metschnikowia* was cultured under an aerobic condition, a marked amount of D-arabitol was produced and accumulated from a fermentable saccharide such as glucose, and thus accomplished the present invention.

That is, the present invention provides a method for producing D-arabitol, which comprises culturing a microorganism belonging to the genus *Metschnikowia* and having D-arabitol producing ability in a medium containing a saccharide as a main carbon source under an aerobic condition, wherein the aerobic condition is achieved by stirring of the medium or aeration of the medium, to produce D-arabitol in the medium. As the saccharide, glucose can be mentioned. As the microorganism belonging to the *Metschnikowia*, *Metschnikowia reukaufii*, *Metschnikowia bicuspidata*, *Metschnikowia lunata* , Metschnikowia pulccherima and *Metschnikowia zobellii* can be mentioned.

The present invention also provides a method for producing D-xylulose, which comprises steps of culturing a microorganism belonging to *the genus Metschnikowia* or a microorganism belonging to the genus Metschnikowia being selected from Metschnikowia pulccherima, Metschnikowia rekaufii, Metschnikowia bicuspidata, Metschnikowia lunata and Metschnikowia zobellii and having D-arabitol producing ability in a medium containing a saccharide as a main carbon source under an aerobic condition, wherein the aerobic condition is achieved by stirring of the medium or aeration of the medium, to produce D-arabitol in the medium, inoculating a microorganism having ability to produce D-xylulose from D-arabitol to the medium, and culturing it to produce D-xylulose in the medium.

The present invention further provides a method for producing xylitol, which comprises steps of culturing a microorganism belonging to the genus *Metschnikowia* or a microorganism belonging to the genus Metschnikowia being selected from Metschnikowia pulccherima, Metschnikowia rekaufii, Metschnikowia bicuspidata, Metschnikowia lunata and Metschnikowia zobellii and having D-arabitol producing ability in a medium containing a saccharide as a main carbon source under an aerobic condition, wherein the aerobic condition is achieved by stirring of the medium or aeration of the medium, to produce D-arabitol in the medium, inoculating a microorganism having ability to produce D-xylulose from D-arabitol to the medium, culturing it to produce D-xylulose in the medium, and allowing a microorganism having ability to convert D-xylulose into xylitol to act on the medium containing D-xylulose, and collecting produced xylitol.

According to the present invention, D-arabitol can be efficiently produced by fermentation using a microbial strain belonging to the genus *Metschnikowia*. Moreover, D-xylulose or xylitol can be produced by using the D-arabitol as a raw material.

### Preferred Embodiments of the Invention

Hereafter, the present invention will be explained in detail.

The microorganism belonging to the genus *Metschnikowia* is not particularly limited so long as it has the ability to produce D-arabitol from a saccharide such as glucose. There can be mentioned, for example, *Metschnikowia pulcherrima*, *Metschnikowia bicuspidata*, *Metschnikowia reukaufii*, *Metschnikowia lunata*, *Metschnikowia zobellii*, *Metschnikowia australis*, *Metschnikowia agaveae*, *Metschnikowia gruessii*, *Metschnikowia hawaiiensis*, *Metschnikowia krissii* and so forth. Among these, *Metschnikowia pulcherrima*, *Metschnikowia reukaufii*, *Metschnikowia bicuspidata, Metschnikowia lunata* and *Metschnikowia zobellii* are preferred. As specific strains, *Metschnikowia pulcherrima* ATCC 18406, *Metschnikowia reukaufii* ATCC 18407, *Metschnikowia bicuspidata* ATCC 24179, *Metschnikowia lunata* ATCC 22033, *Metschnikowia zobellii* ATCC 22302 and *Metschnikowia pulcherrima* FERM BP-7161 can be mentioned. These strains can be obtained from American Type Culture Collection, Address: 12301 Parklawn Drive, Rockville, Maryland 20852, and United States of America. *Metschnikowia pulcherrima* FERM BP-7161 was originally deposited at the National Institute of Bioscience and Human-Technology, Agency of Industrial Science and Technology, Ministry of International Trade and Industry (postal code: 305-8566, 1-3 Higashi 1-Chome, Tsukuba-shi, Ibaraki-ken, Japan) on January 16, 1998, under the deposition number of FERM P-16592 and transferred from the original deposit to international deposit based on Budapest Treaty on May 15, 2000, and has been deposited as deposition number of FERM BP-7161.

By culturing such a microorganism belonging to the genus *Metschnikowia* as mentioned above in a medium containing saccharide as a main carbon source under an aerobic condition, D-arabitol can be produced in the medium. While one kind of the microorganism belonging to the genus *Metschnikowia* can be used, arbitrary two or more kinds of such microorganisms can be used simultaneously.

In present invention, a liquid medium is usually used as the medium. The saccharide is not particularly limited so long as D-arabitol is produced when it is used for the culture of the microorganism belonging to the genus *Metschnikowia* under an aerobic condition. For example, there can be mentioned glucose, fructose, sucrose and so forth. As for the amount of these saccharides in the medium, they are desirably used in an amount in the range of 5-40 g/dl. The saccharide may be added stepwise during the culture.

As a nitrogen source, a nitrogen compound which can be utilized by the microorganism, for example, ammonium sulfate, yeast extract, peptone, malt extract, casamino acid, corn steep liquor and so forth may be used. Further, as inorganic salts added to the medium, for example, salts such as magnesium sulfate, potassium dihydrogenphosphate, dipotassium hydrogenphosphate and potassium hydroxide may be used. Furthermore, various kinds of organic substances and inorganic substances required for growth of the microorganism, surface active agents, anti-foaming agents usually used can be added as required. Medium components such as nitrogen sources, mineral salts and other nutrients can also be added as required.

The microorganism belonging to the genus *Metschnikowia* of the present invention is cultured under an aerobic condition. The term "aerobic condition" means that the culture is performed at an oxygen concentration in the medium higher than that obtained in stationary culture. The oxygen concentration is preferably controlled so that dissolved oxygen concentration should be represented as a positive value over the whole culture period, and more preferably it is controlled so that oxygen partial pressure should be 0.05 to 0.1 atm. Such an oxygen concentration may be attained by stirring of the medium on aeration

Inoculation of the microorganism belonging to the genus *Metschnikowia* is performed, for example, by directly inoculating cells grown on a slant medium containing 2 g/dl of D-glucose, 1 g/dl of yeast extract, 1 g/dl of malt extract and 2 g/dl of agar, pH 7.0, at 30°C for 24 hours into a production medium, or by inoculating a seed culture broth which has been separately obtained by preculture. The seed culture broth is prepared by, for example, inoculating one platinum loop of the aforementioned cells cultured on the slant medium into a sterilized culture broth containing 5 g/dl of glucose, 0.3 g/dl of yeast extract, 0.15 g/dl of ammonium sulfate, 0.1 g/dl of potassium dihydrogenphosphate, 0.3 g/dl of dipotassium hydrogenphosphate, 0.1 g of magnesium sulfate heptahydrate and 2.5 g/dl of calcium carbonate (separately sterilized), pH 5-7, and culturing them at a temperature of 30-34°C for 12-24 hours.

Culture temperature of the main culture should be within the range where the microorganism belonging to the genus *Metschnikowia* can grow. That is, the culture is performed usually at a temperature within the range of 25-37°C, preferably in the range of 30-34°C. Further, pH of the medium is usually controlled to be within the range of 3-9, preferably 4-7. While culture period may vary depending on kind of the medium to be used and the concentration of the saccharide that is the main carbon source of the medium, it is usually about 12 hours to 10 days. The culture according to the present invention is preferably terminated when the nutritional sources in the medium are utilized to a maximum extent, and the accumulated amount of D-arabitol produced in the medium reaches a maximum value.

The amount of the produced D-arabitol in the culture broth can be quickly measured by using a known technique such as high performance liquid chromatography. D-arabitol accumulated in the culture broth as described above may be collected from the culture broth in a conventional manner, or it may be used in the culture broth as it is when it is used as a substrate to be converted into another substance. D-arabitol is collected from the culture broth by a suitable combination of well known techniques commonly used in this field such as filtration, centrifugation, vacuum concentration, ion exchange or adsorption chromatography, solvent extraction, distillation and crystallization as required. For example, cells are removed from the culture broth by filtration or centrifugation, and the medium is treated with activated carbon to remove coloring substances and so forth, deionized by using an ion exchange resin, and concentrated under vacuum to form a syrup. Then, the syrup can be extracted with an alcohol, and crystals can be deposited from the extract, and recrystallized from an alcohol to obtain pure crystals.

By using D-arabitol produced and accumulated in the medium or collected as described above as a raw material, D-xylulose or xylitol can be produced. That is, D-xylulose can be produced by allowing a microorganism having the ability to produce D-xylulose from D-arabitol to act on D-arabitol or the culture broth containing D-arabitol.

For example, as described in *Applied Microbiology*, *18*, 1031-1035 (1969), D-arabitol produced by fermentation can be converted into D-xylulose by allowing *Acetobacter suboxydans* to act on the D-arabitol. Alternatively, as disclosed in EP 403 392A and EP 421 882A, D-arabitol produced by fermentation can be converted into D-xylulose by allowing an *Acetobacter* bacterium, *Gluconobacter* bacterium or *Klebsiella* bacterium to act on the D-arabitol. As a specific example of such a bacterial strain, *Gluconobacter oxydans* ATCC 621 strain can be mentioned.

As other microorganisms having the ability to convert D-arabitol into D-xylulose, there can be mentioned microorganisms belonging to the genera *Achromobacter, Agrobacterium*, *Alcaligenes, Arthrobacter, Aureobacterium*, *Azotobacter*, *Brevibacterium*, *Corynebacterium, Enterobacter*, *Erwinia*, *Flavobacterium*, *Micrococcus, Morganella, Nocardia, Planococcus,* Proteus, *Propionibacterium, Pseudomonas, Rhodococcus*, *Sporosarcina, Staphylococcus*, *Vibrio*, *Actinomadura*, *Actinomyces*, *Kitasatosporia, Streptomyces*, *Aeromonas*, *Aureobacterium*, *Bacillus, Escherichia*, *Microbacterium*, *Serratia*, *Salmonella* and *Xanthomonas*.

D-xylulose can also be produced by inoculating such a microorganism having the ability to convert D-arabitol into D-xylulose as mentioned above into a culture broth of *Metschnikowia* yeast in which D-arabitol is produced or such a culture broth from which *Metschnikowia* yeast cells are removed, and culturing the microorganism.

Further, xylitol can be produced by allowing a microorganism having the ability to convert D-xylulose into xylitol to act on D-xylulose produced as described above. As the microorganism having the ability to convert D-xylulose into xylitol, *Candida guilliermondii* var. soya (*Applied Microbiology*, *18*, 1031-1035 (1969)) has been known.

Furthermore, xylitol can also be produced by allowing a microorganism transformed with a xylitol dehydrogenase gene and having ability to supply reducing power on D-xylulose. The term "ability to supply reducing power" used herein means an ability to supply NADH (reduced type nicotinamide adenine dinucleotide) in an amount sufficient for allowing proceeding of the reduction reaction of D-xylulose into xylitol catalyzed by xylitol dehydrogenase (D-xylulose reductase). As specific examples of microorganisms having such ability, *Escherichia* bacteria such as *Escherichia coli* can be mentioned.

Alternatively, as disclosed in EP 403 392A and EP 421 882A, the produced D-xylulose can be converted into D-xylose by allowing glucose isomerase (xylose isomerase) to act on the D-xylulose, and then the produced D-xylose can be hydrogenated to be converted into xylitol.

Moreover, it is also possible to produce xylitol from D-arabitol produced by using *Metschnikowia* yeast by using a microorganism belonging to the genus *Gluconobacter* or *Acetobacter* and having an ability to convert D-arabitol into xylitol (WO 99/20782).

The methods for producing D-xylulose or xylitol from D-arabitol are not limited to those mentioned above, and any known methods may be used. Moreover, even a method that has not been known so far may also be used for the present invention.

### Best Mode for Carrying out the Invention

Hereafter, the present invention will be explained more specifically with reference to the following examples.

### Example 1: Production of D-arabitol

D-Arabitol was produced by using the *Metschnikowia pulcherrima* ATCC 18406 strain, *Metschnikowia bicuspidata* ATCC 24179 strain, *Metschnikowia reukaufii* ATCC 18407 strain, *Metschnikowia lunata* ATCC 22033 strain, *Metschnikowia zobellii* ATCC 22302 strain and *Metschnikowia pulcherrima* FERM BP-7161 strain as microorganisms belonging to the genus *Metschnikowia*.

Each of the aforementioned strains was cultured on a slant medium containing 2 g/dl of D-glucose, 1 g/dl of yeast extract, 1 g/dl of malt extract and 2 g/dl of agar, pH 7.0, at 30°C for 24 hours, and one platinum loop of the cells were inoculated into 20 ml of seed culture medium (5 g/dl of D-glucose, 0.3 g/dl of yeast extract, 0.15 g of ammonium sulfate, 0.1 g/dl of potassium dihydrogenphosphate, 0.3 g/dl of dipotassium hydrogenphosphate, 0.1 g/dl of magnesium sulfate heptahydrate and 2.5 g/dl of calcium carbonate (separately sterilized), pH 7.0, sterilized at 120°C for 20 minutes) contained in a 500-ml Sakaguchi flask, and cultured at 30°C for 18 hours with shaking at 120 rpm. The obtained culture medium was used as seed culture broth.

One milliliter of the seed culture broth was inoculated into 20 ml of main culture medium (10 g/dl of D-glucose, 0.5 g/dl of yeast extract, 0.15 g/dl of ammonium sulfate, 0.1 g/dl of potassium dihydrogenphosphate, 0.3 g/dl of dipotassium hydrogenphosphate, 0.1 g/dl of magnesium sulfate heptahydrate and 2.5 g/dl of calcium carbonate (separately sterilized), pH 7.0, sterilized at 120°C for 20 minutes) contained in a 500-ml Sakaguchi flask, and cultured at 30°C with shaking at 120 rpm.

Concentrations of the formed D-arabitol and residual D-glucose in the culture broth were periodically measured. As for the measurement of D-arabitol concentration, cells were removed from each collected portion of the culture broth by centrifugation, and the supernatant was suitably diluted and subjected to high performance liquid chromatography to measure the D-arabitol concentration. The high performance liquid chromatography was performed by using Shodex Sugar SC1211 as a column and 50% (v/v) acetonitrile containing 12.5 mg/L of calcium nitrate and 12.5 mg/L of EDTA as a mobile phase at a column temperature of 60°C and a flow rate of 0.8 ml/min. The results of the analysis are as shown in Table 1.

**Table 1**

| Fermentative Production of D-arabitol from D-glucose by *Metschnikowia* yeast | | | | |
|---|---|---|---|---|
| Strain | Saccharide concentration at time 0 (g/dl) | Culture time (hr) | Concentration of accumulated D-arabitol (g/dl) | Residual saccharide (g/dl) |
| *Metschnikowia pulcherrima* ATCC 18406 | 9.5 | 54 | 1.3 | 0.0 |
| *Metschnikowia reukaufii* ATCC18407 | 9.5 | 54 | 2.1 | 0.0 |
| *Metschnikowia bicuspidata* ATCC 24179 | 9.5 | 54 | 2.6 | 0.0 |
| *Metschnikowia lunata* ATCC 22033 | 9.5 | 54 | 2.7 | 0.0 |
| *Metschnikowia zobellii* ATCC 22302 | 9.5 | 30 | 0.7 | 2.7 |
| *Metschnikowia pulcherrima* FERM BP-7161 | 9.5 | 30 | 1.7 | 0.0 |

As shown in Table 1, D-arabitol was formed by all of the used *Metschnikowia* yeast strains, and the weight yield based on the consumed saccharide (ratio of weight of the formed D-arabitol to weight of the consumed saccharide) reached 28% for the highest value.

Cells were removed by centrifugation from each culture broth obtained as described above, cations and anions were removed from the culture broth successively by using a cation exchange resin and anion exchange resin, respectively. The obtained solution was concentrated under vacuum to substantially completely eliminate the moisture, and extracted with ethanol. The extract was crystallized, and the crystals were recrystallized from ethanol to obtain pure crystals of D-arabitol.

### Example 2: Production of D-xylulose

Culture broth containing D-arabitol was obtained in the same manner as in Example 1 by using the *Metschnikowia lunata* ATCC 22033 strain, and the cells were removed from this culture broth by centrifugation to obtain culture broth containing 2.7 g/dl of D-arabitol.

Then, the *Gluconobacter oxydans* ATCC 621 strain was inoculated into 3 ml of a sterilized medium (pH 6.0) containing 2.4 g/dl of potato dextrose, 3 g/dl of yeast extract, 0.5 g/dl of glycerol, 3 g/dl of mannitol and 2 g/dl of calcium carbonate (separately sterilized) contained in a test tube, and cultured at 30°C for 16 hours.

The above culture broth of *Gluconobacter oxydans* was inoculated at a concentration of 5% into the aforementioned culture broth containing D-arabitol, and cultured at 30°C with shaking. After 17 hours, the culture was terminated, and cells were removed by centrifugation. As a result, 2.3 g/dl of D-xylulose was obtained from 2.5 g/dl of D-arabitol at the time of inoculation (yield: 92%).

### Example 3: Production of xylitol

### (1) Preparation of Escherichia coli transformed with xylitol dehydrogenase gene

Based on the known nucleotide sequence of xylitol dehydrogenase gene derived from *Morganella morganii* ATCC 25829 (DDBJ/GenBank/EMBL accession No. L34345), oligonucleotides having the nucleotide sequences shown as SEQ ID NOS: 1 and 2 in Sequence Listing were synthesized.

PCR was performed by using the synthesized oligonucleotides as primers and genomic DNA of the strain as a template under usual conditions. As a result, a DNA fragment of about 1.2 kb containing xylitol dehydrogenase gene was amplified. This DNA fragment was separated and collected by agarose gel electrophoresis, digested with EcoRI and BamHI for the both ends, and ligated to an EcoRI and BamHI digestion product of pUC18 (Takara Shuzo). *Escherichia coli* JM109 was transformed with the ligation product, and a transformed strain containing the xylitol dehydrogenase gene was selected. If the cloned fragment contained the xylitol dehydrogenase gene, the gene would be expressed as a fusion gene with the lacZ' gene under control by lac promoter.

The xylitol dehydrogenase activity of the transformed strain was measured as follows. The cells cultured overnight in L medium (1% trypton, 0.5% yeast extract, 0.5% NaCl) were disrupted by sonication, and the supernatant was used as a crude enzyme solution. The reaction was performed in 1 ml of a reaction mixture containing of 100 mM glycine buffer (pH 9.5), 100 mM xylitol, 2 mM NAD and 100 µl of the crude enzyme solution at 30°C for 1 minute. Formation of NADH or NADPH was detected based on increase of absorbance at 340 nm. The enzymatic activity oxidizing 1 µmol of xylitol to produce 1 µmol of NADH in 1 minute under the aforementioned conditions was defined as one unit.

As a result, 3.9 U/mg of xylitol dehydrogenase activity was observed in the transformed strain, whereas the enzymatic activity was not observed in *Escherichia coli* JM109 (host strain) used as a control. Thus, it was confirmed that the transformed strain had the xylitol dehydrogenase gene.

### (2) Production of xylitol using Escherichia coli transformed with xylitol dehydrogenase gene

The transformed strain obtained above was inoculated into 50 ml of a medium (pH 6.0) containing 0.5 g/dl of yeast extract, 0.5 g/dl of peptone, 0.5 g/dl of beef extract, 0.5 g/dl of ammonium sulfate, 0.1 g/dl of potassium dihydrogenphosphate, 0.3 g/dl of dipotassium hydrogenphosphate, 0.05 g/dl of magnesium sulfate heptahydrate, 0.001 g/dl of iron sulfate heptahydrate, 0.001 g/dl of manganese sulfate n-hydrate, 1 g/dl of glycerol and 2 g/dl of calcium carbonate (separately sterilized) contained in a 500-ml volume flask, and cultured at 30°C for 3 hours with shaking. To this culture, isopropyl-β-D-thiogalactopyranoside (IPTG) was added at a final concentration of 1 mM, and further cultured for 4 hours.

The culture supernatant of *Gluconobacter oxydans* containing D-xylulose obtained in Example 2 was added with the culture broth obtained as described above at a final concentration of 5%, and further added with D-glucose at a final concentration of 1 g/dl. Then, culture was carried out at 30°C with shaking. As a result, 2.1 g/dl of xylitol was obtained from 2.2 g/dl of D-xylulose at the time of starting the culture (yield: 95%) by culture for 24 hours.

## Claims

1. A method for producing D-arabitol, which comprises culturing a microorganism belonging to the genus Metschnikowia and having D-arabitol producing ability in a medium containing a saccharide as a main carbon source under an aerobic condition, wherein the aerobic condition is achieved by stirring of the medium or aeration of the medium.

2. The method according to claim 1, wherein the saccharide is glucose.

3. The method according to claim 1, wherein the microorganism belonging to the genus Metschnikowia is selected from Metschnikowia pulccherima, Metschnikowia rekaufii, Metschnikowia bicuspidata, Metschnikowia lunata and Metschnikowia zobellii.

4. A method for producing D-xylulose, which comprises the steps of:
culturing a microorganism belonging to the genns Metschnikowia or a microorganism belonging to the genns Metschnikowia being selected from Metschnikowia pulccharima, Metschnikowia rekantii, Metschnikowia biscuspidata, Metschnikowia lunata and Metschnikowia zobellii and having O-arabitol producing ability, or having the accession number FERM BP-7161, under an aerobic condition, wherein the aerobic condition is achieved by stirring of the medium or aeration of the medium,
inoculating a microorganism having the ability to produce D-xylulose from D-arabitol to the medium, and
culturing it.

5. A method for producing D-xylitol, which comprises the steps of:
culturing a microorganism belonging to the genns Metschnikowia or a microorganism belonging to the genns Metschnikowia being selected from Metschnikowia pulccharima, Metschnikowia rekantii, Metschnikowia biscuspidata, Metschnikowia lunata and Metschnikowia zobellii and having O-arabitol producing ability, or having the accession number FERM BP-7161, under an aerobic condition, wherein the aerobic condition is achieved by stirring of the medium or aeration of the medium
inoculating a microorganism having the ability to produce D-xylulose from D-arabitol to the medium, culturing it to produce D-xylulose and
allowing a microorganism having the ability to convert D-xylulose into xylitol to act on D-xylulose.

6. The method for producing D-arabitol according to claim 1, wherein an oxygen concentration in the medium is higher than that in stationary culture.

## Patentansprüche

1. Verfahren zur Herstellung von D-Arabitol, welches das Kultivieren eines Mikroorganismus der Gattung Metschnikowia mit der Fähigkeit zur Produktion von D-Arabitol in einem Medium, das ein Saccharid als Hauptkohlenstoffquelle enthält, unter aeroben Bedingungen umfaßt, wobei die aeroben Bedingungen durch Rühren des Mediums oder Belüftung des Mediums erreicht werden.

2. Verfahren nach Anspruch 1, wobei das Saccharid Glucose ist.

3. Verfahren nach Anspruch 1, wobei der Mikroorganismus der Gattung Metschnikovia unter Metschnikovia pulccherima, Metschnikovia rekaufii, Metschnikovia bicuspidata, Metschnikovia lunata und Metschnikovia zobellii ausgewählt ist.

4. Verfahren zur Herstellung von D-Xylulose, welches die folgenden Stufen umfaßt:
Kultivieren eines Mikroorganismus der Gattung Metschnikovia oder eines Mikroorganismus der Gattung Metschnikovia, der unter Metschnikovia pulccherima, Metschnikovia rekaufii, Metschnikovia bicuspidata, Metschnikovia lunata und Metschnikovia zobellii ausgewählt ist und die Fähigkeit zur Produktion von D-Arabitol hat, oder eines Mikroorganismus mit der Hinterlegungsnummer FERM BP-7161 unter aeroben Bedingungen, wobei die aeroben Bedingungen durch Rühren des Mediums oder Belüften des Mediums erreicht werden,
Animpfen des Mediums mit einem Mikroorganismus mit der Fähigkeit zur Produktion von D-Xylulose aus D-Arabitol und
Kultivieren des Mikroorganismus.

5. Verfahren zur Herstellung von D-Xylitol, welches die folgenden Stufen umfaßt:
Kultivieren eines Mikroorganismus der Gattung Metschnikovia oder eines Mikroorganismus der Gattung Metschnikovia, der unter Metschnikovia pulccherima, Metschnikovia rekaufii, Metschnikovia bicuspidata, Metschnikovia lunata und Metschnikovia zobellii ausgewählt ist und die Fähigkeit zur Produktion von D-Arabitol hat, oder eines Mikroorganismus mit der Hinterlegungsnummer FERM BP-7161 unter aeroben Bedingungen, wobei die aeroben Bedingungen durch Rühren des Mediums oder Belüftung des Mediums erreicht werden
Animpfen des Mediums mit einem Mikroorganismus mit der Fähigkeit zur Produktion von D-Xylulose aus D-Arabitol,
Kultivieren des Mikroorganismus zur Produktion von D-Xylulose und
Einwirkenlassen eines Mikroorganismus mit der Fähigkeit, D-Xylulose in Xylitol umzuwandeln, auf D-Xylulose.

6. Verfahren zur Herstellung von D-Arabitol nach Anspruch 1, wobei die Sauerstoffkonzentration in dem Medium höher als in stationärer Kultur ist.

## Revendications

1. Méthode pour la production du D-arabitol, qui comprend la culture d'un microorganisme appartenant au genre *Metschnikowia* et ayant la capacité de produire du D-arabitol dans un milieu contenant un saccharide en tant que source principale de carbone en condition aérobie, dans laquelle la condition aérobie est obtenue par l'agitation du milieu ou l'aération du milieu.

2. Méthode selon la revendication 1, dans laquelle le saccharide est le glucose.

3. Méthode selon la revendication 1, dans laquelle le microorganisme appartenant au genre *Metschnikowia* est choisi parmi *Metschnikowia pulccherima, Metschnikowia rekaufii, Metschnikowia bicuspidata, Metschnikowia lunata* et *Metschnikowia zobellii.*

4. Méthode pour la production de D-xylulose, qui comprend les étapes de :
culture d'un microorganisme appartenant au genre *Metschnikowia* ou d'un microorganisme appartenant au genre *Metschnikowia* choisi parmi *Metschnikowia pulccherima, Metschnikowia rekaufii, Metschnikowia bicuspidata, Metschnikowia lunata* et *Metschnikowia zobellii* et ayant la capacité de produire du D-arabitol, ou ayant le numéro d'enregistrement FERM BP-7161, en condition aérobie, dans laquelle la condition aérobie est obtenue par l'agitation du milieu ou l'aération du milieu,
ensemencement du milieu par un microorganisme ayant la capacité de produire du D-xylulose à partir du D-arabitol et culture de ce dernier.

5. Méthode pour la production de D-xylitol, qui comprend les étapes de :
culture d'un microorganisme appartenant au genre *Metschnikowia* ou d'un microorganisme appartenant au genre *Metschnikowia* choisi parmi *Metschnikowia pulccherima, Metschnikowia rekaufii, Metschnikowia bicuspidata, Metschnikowia lunata* et *Metschnikowia zobellii* et ayant la capacité de produire du D-arabitol, ou ayant le numéro d'enregistrement FERM BP-7161, en condition aérobie, dans laquelle la condition aérobie est obtenue par l'agitation du milieu ou l'aération du milieu,
ensemencement du milieu par un microorganisme ayant la capacité de produire du D-xylulose à partir du D-arabitol, culture de ce dernier pour produire du D-xylulose et
action, sur le D-xylulose, d'un microorganisme ayant la capacité de convertir le D-xylulose en xylitol.

6. Méthode pour la production de D-arabitol selon la revendication 1, dans laquelle la concentration de l'oxygène dans le milieu est supérieure à celle en culture stationnaire.
